# EUROPÄISCHE PATENTANMELDUNG

(11) **EP 1 783 295 A2**
(43) Veröffentlichungstag der Anmeldung: **09.05.2007**
(21) Anmeldenummer: 06022601.6
(22) Anmeldetag: 30.10.2006
(51) Int. Cl.: E04C 5/04, E04C 5/07, E04F 13/04, A61N 1/16, H05K 9/00, E04B 1/92

(54) **Armierung aus Fäden oder Fasern**

(30) Priorität: 02.11.2005 DE 202005017236 U
(71) Anmelder: Vitrulan Textilglas GmbH, 95509 Marktschorgast (DE); Kollmann, Helga, 8664 Veitsch (AT); Kollmann, Wolfgang, 8664 Veitsch (AT)
(72) Erfinder: Kollmann, Wolfgang, 8664 Veitsch (AT); Dorsch, Wolfgang, 95509 Marktschorgast (DE)
(74) Vertreter: Blaumeier, Jörg

(57) **Zusammenfassung**

Armierung für Wärmedämmverbundsysteme oder zum Einbau in Bauwerksfassaden, -dächer, -verkleidungen oder -wände, bestehend aus mit Appretur versehenen Fäden oder Fasern, wobei die Armierung (1) zumindest halbseitig mit einer metallischen, elektrisch leitfähigen, durch ein Metallisierungsverfahren aufgebrachten Beschichtung (4) versehen ist.

## Beschreibung

Die Erfindung betrifft eine Armierung für Wärmedämmverbundsysteme oder zum Einbau in Bauwerksfassaden, -dächer, -verkleidungen oder -wände, bestehend aus mit Appretur versehenen Fäden oder Fasern.

Armierungen dieser Art werden beispielsweise für das Verputzen von Gebäuden, die Stabilisierung von Bauteilen oder die Aufnahme thermohygrischer Spannungen bei Wärmedämmverbundsystemen verwendet. Beim Verputzen von Gebäuden werden sie meistens in Matten oder Bahnen entlang des zu verputzenden Bereiches angebracht. Durch die Armierung wird die Rissbildung in den Putzen und Putzsystemen reduziert.

Mit der zunehmenden Zahl von Quellen elektromagnetischer Felder in der Nähe von Wohnbereichen kommt auch die Befürchtung auf, dass diese elektromagnetischen Felder (sog. Elektrosmog) negative Auswirkungen auf die Gesundheit des Menschen haben könnten. Dementsprechend wird beim Bau eines Hauses häufig der Wunsch geäußert, einen Schutz gegen elektromagnetische Strahlung im Inneren des Hauses zu erreichen. Dies gilt insbesondere in der Nähe von Hochspannungsleitungen, Straßenbahn-/Eisenbahnoberleitungen, Rundfunk- und Fernsehmasten sowie Mobiltelefonsendern.

Durch eine elektrisch leitende Ausgestaltung der Armierungen, die ohnehin beim Bau der Gebäude verwendet werden, lässt sich eine Art faradayscher Käfig um die Wohnräume herum erzeugen, der vor elektromagnetischer Strahlung schützt.

Eine solche Armierung ist in der DE 199 42 882 A1 beschrieben. Dort sind sich kreuzende Fäden vorgesehen, die Metallseelen aufweisen. Um einen guten elektrisch leitenden Anschluss an weitere Matten bzw. Bahnen zu erreichen, ist zusätzlich vorgesehen, den eine Appretur aufweisenden Fäden leitfähige Substanzen, wie beispielsweise Kohlenstoff, zuzufügen. Allerdings sind die Metallseelen nur in aufwändigen Herstellungsprozessen in die Fäden einflechtbar bzw. mit den Fäden verwebbar und es ist zusätzlich eine Modifikation der Appretur erforderlich.

Der Erfindung liegt daher die Aufgabe zugrunde, eine Armierung zu schaffen, die einfach herstellbar ist und eine gute Abschirmung gegen elektromagnetische Wellen ermöglicht.

Zur Lösung dieser Aufgabe ist bei einer Armierung der eingangs genannten Art erfindungsgemäß vorgesehen, dass die Armierung zumindest halbseitig mit einer metallischen, elektrisch leitfähigen, durch ein Metallisierungsverfahren aufgebrachten Beschichtung versehen ist.

Im Rahmen der vorliegenden Erfindung ist auf eine Armierung zumindest halbseitig durch ein Metallisierungsverfahren eine Metallbeschichtung aufgebracht. Die übrigen Herstellungsschritte können dabei vorteilhafterweise auf dieselbe Art und Weise wie bei herkömmlichen Armierungen erfolgen, die keinen Schutz gegen elektromagnetische Felder und Wellen bieten sollen. Als weiterer Herstellungsschritt wird dann auf die Armierung die metallische Beschichtung aufgebracht.

Die zusätzliche Beschichtung ist untrennbar mit der auf die Fäden oder Fasern aufgebrachten Appretur verbunden. Jedoch werden dadurch die für die Anwendung relevanten Eigenschaften beispielsweise Flexibilität, Verarbeitbarkeit, Affinität zu handelsüblichen Putzen und Klebern nicht verschlechtert.

Als Metallisierungsverfahren kann ein einziges Verfahren oder eine Kombination mehrerer nacheinander durchgeführter Verfahren vorgesehen sein, hierzu bieten sich insbesondere galvanische Verfahren (stromunterstützte und außenstromlose elektrolytische Abscheidung), physikalische oder chemische Beschichtungsverfahren aus der Gasphase und thermisches Spritzen an. Die galvanischen, also elektrochemischen, Verfahren werden dabei bevorzugt.

Die Armierung kann auch beidseitig mit der Beschichtung versehen sein, vorzugsweise kann die Oberfläche vollständig mit dem abgeschiedenen Metall oder einer Metalllegierung überzogen sein. Durch die zusammenhängende, sich über die gesamte Fläche der Armierung erstreckende leitfähige Beschichtung ist zudem gewährleistet, dass bei Anschluss zweier verschiedener Matten oder Bahnen aneinander eine elektrisch leitfähige Verbindung gegeben ist.

Vorteilhafterweise kann die Beschichtung aus Kupfer bestehen. Kupfer hat sich aufgrund seiner Eigenschaften als das am besten geeignete Metall erwiesen.

In weiterer Ausgestaltung der Erfindung kann die Beschichtung von einer insbesondere alkaliresistenten Korrosionsschutzschicht umgeben sein. Die Korrosionsschutzschicht kann Ni, Co, NiP, NiCoP, CoP, NiW, NiMo, NiSn, NiZn, NiCr, TiN, Ti, CnZn, CnSn oder CrN oder eine Kombination oder eine Legierung davon umfassen. Eine solche Schutzschicht verhindert, dass die Metallschicht und bei vollständiger Metallisierung auch die Fasern durch Wasser oder sonstige Einflüsse, insbesondere alkalische Einflüsse, angegriffen werden. Mit besonderem Vorteil kann die Korrosionsschutzschicht selbst elektrisch leitfähig ausgebildet sein, wodurch eine elektrisch leitfähige Verbindung auch am Übergang zwischen verschiedenen Matten oder Bahnen gewährleistet ist.

Die Fäden oder Fasern können im Rahmen der Herstellung auf verschiedene Arten zu einer Armierung verarbeitet sein. So kann die Armierung als Gewebe oder Gelege oder Gewirke oder als Gittermatte ausgebildet sein. Insbesondere ein Gewebe mit einer Dreherbindung hat sich als zweckmäßig erwiesen. Fäden aus mehreren Fasern können alternativ auch übereinander gelegt und an den Kreuzungspunkten miteinander verbunden sein. Ein besonderes Augenmerk ist hierbei auf die Maschenweite zu legen. Erfindungsgemäß kann eine Maschenweite von 2 mm bis 8 mm, insbesondere 4 mm, vorgesehen sein. Damit werden sowohl die Anforderungen an die abzuschirmende Hochfrequenz erfüllt, durch die die Maschenweite nach oben begrenzt wird, wie auch die Anforderungen an die Haltekraft des Putzes, durch die die Maschenweite nach unten begrenzt wird. Als ideal haben sich Maschen von 4 mal 4 mm erwiesen.

Die Fäden können aus einzelnen Fasern gebildete Bündel sein. Als Material für die Fäden sind mineralische, synthetische oder native Fasern geeignet, von diesen haben sich Glasfasern als besonders zweckmäßig erwiesen.

Weitere Vorteile und Einzelheiten der Erfindung ergeben sich aus dem im Folgenden beschriebenen Ausführungsbeispiel sowie anhand der Zeichnungen. Dabei zeigen:
- Fig. 1: eine erfindungsgemäße Armierung, und
- Fig. 2: den Querschnitt eines Fadens der erfindungsgemäßen Armierung.

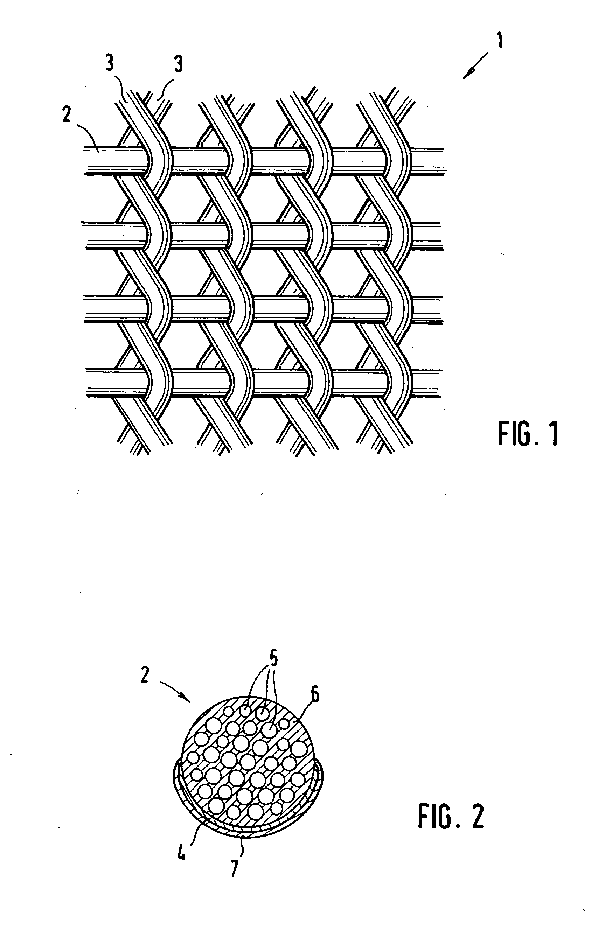

Wie in Fig. 1 gezeigt ist, besteht die Armierung 1 aus horizontal verlaufenden Fäden 2 sowie im Wesentlichen vertikal verlaufenden Fäden 3, die ein Gewebe mit einer Dreherbindung bilden. Die Maschenweite beträgt etwa 4 mal 4 mm. Die Fäden 2 und 3 umfassen jeweils ein Bündel von mehreren Glasfasern. Die Armierung 1 ist halbseitig mit einer metallischen, galvanisch aufgebrachten Beschichtung 4 aus Kupfer oder einer artverwandten Legierung versehen. Diese ist elektrisch leitfähig, so dass bei bestimmungsgemäßer Anbringung des Gewebes in der Gebäudehülle die Stärke elektromagnetischer Felder in Gebäuden reduziert werden kann.

Fig. 2 zeigt den Querschnitt eines Fadens 2. Dieser besteht aus mehreren Filamenten oder Glasfasern 5. Die Glasfasern 5 sind von einer Appretur 6 umgeben. Die Appretur 6 wirkt als Ummantelung und ist in dem dargestellten Ausführungsbeispiel eine Kunststoffverbindung. Der Faden 2 - wie auch die ganze Armierung 1 - ist halbseitig galvanisiert, das bedeutet, es ist halbseitig die metallische, elektrisch leitende Beschichtung 4 aus Kupfer vorgesehen. Die Beschichtung 4 ist von einer diese umschließenden Korrosionsschutzschicht 7 ummantelt, die die Korrosion der Beschichtung 4 verhindert und sie beispielsweise vor alkalischen Einflüssen schützt. Auch die Korrosionsschutzschicht 7 ist elektrisch leitfähig, so dass bei überlappendem Einbau in Verbindung mit der so genannten gestürzten Verlegung eine leitfähige Verbindung zwischen einzelnen Gewebebahnen hergestellt werden kann. Bei der Herstellung kann sich die Korrosionsschutzschicht 7 nur auf dem Kupfer verankern, so dass nur die Beschichtung 4 überdeckt ist, nicht jedoch zum Beispiel der in Fig. 2 gezeigte obere Bereich des Fadens 2.

Die Armierung 1 aus Glasfasern 5, die mit der Appretur 6 versehen ist, hat ein Gesamtgewicht von etwa 50 - 400 g/m², insbesondere 120 - 160 g/m², davon beträgt der Anteil der Appretur 6 ca. 10 - 40 %, insbesondere 20 %. Die zusätzliche Beschichtung 4 und die Korrosionsschutzschicht 7 wird bei einseitiger Applikation mit ca. 14 bis 22 g/m² auf das Gewebe aufgebracht.

## Patentansprüche

1. Armierung für Wärmedämmverbundsysteme oder zum Einbau in Bauwerksfassaden, -dächer, -verkleidungen oder -wände, bestehend aus mit Appretur versehenen Fäden oder Fasern, **dadurch gekennzeichnet, dass** die Armierung (1) zumindest halbseitig mit einer metallischen, elektrisch leitfähigen, durch ein Metallisierungsverfahren aufgebrachten Beschichtung (4) versehen ist.

2. Armierung nach Anspruch 1, **dadurch gekennzeichnet, dass** das Metallisierungsverfahren ein Verfahren oder eine Kombination von Verfahren aus der Gruppe, umfassend galvanische Verfahren, physikalische oder chemische Beschichtungsverfahren aus der Gasphase und thermisches Spritzen, umfasst.

3. Armierung nach Anspruch 1 oder 2, **dadurch gekennzeichnet, dass** sie beidseitig, vorzugsweise vollständig, mit der Beschichtung (4) versehen ist.

4. Armierung nach einem der vorangehenden Ansprüche, **dadurch gekennzeichnet, dass** die Beschichtung (4) aus Kupfer oder einer artverwandten Legierung besteht.

5. Armierung nach einem der vorangehenden Ansprüche, **dadurch gekennzeichnet, dass** die Beschichtung (4) von einer insbesondere alkaliresistenten Korrosionsschutzschicht umgeben ist.

6. Armierung nach Anspruch 5, **dadurch gekennzeichnet, dass** die Korrosionsschutzschicht Ni, Co, NiP, NiCoP, CoP, NiW, NiMo, NiSn, NiZn, NiCr, TiN, Ti, CnZn, CnSn oder CrN oder eine Kombination oder eine Legierung davon umfasst.

7. Armierung nach Anspruch 5 oder 6, **dadurch gekennzeichnet, dass** die Korrosionsschutzschicht elektrisch leitfähig ist.

8. Armierung nach einem der vorangehenden Ansprüche, **dadurch gekennzeichnet, dass** sie als Gewebe oder Gelege oder Gewirke oder als Gittermatte ausgebildet ist.

9. Armierung nach Anspruch 8, **dadurch gekennzeichnet, dass** die Maschenweite des Gewebes oder Gewirkes 2 bis 8 mm, insbesondere 4 mm, beträgt.

10. Armierung nach einem der Ansprüche 8 oder 9, **dadurch gekennzeichnet, dass** das Gewebe eine Dreherbindung aufweist.

11. Armierung nach einem der vorangehenden Ansprüche, **dadurch gekennzeichnet, dass** die Fäden (3) aus einzelnen Fasern bestehende Bündel sind.

12. Armierung nach einem der vorangehenden Ansprüche, **dadurch gekennzeichnet, dass** die Fasern mineralische, synthetische oder native Fasern, insbesondere Glasfasern (5) oder Kunststofffasern, sind.

13. Armierung nach einem der vorangehenden Ansprüche, **dadurch gekennzeichnet, dass** der Gewichtsanteil der Appretur etwa 10 - 40 %, vorzugsweise etwa 20 %, beträgt, bezogen auf das Gesamtgewicht der Armierung.

14. Armierung nach einem der vorangehenden Ansprüche, **dadurch gekennzeichnet, dass** sie ein Gesamtgewicht von etwa 50 - 400 g/m², insbesondere 120 - 160 g/m², aufweist.
